# EUROPEAN PATENT SPECIFICATION

(11) **EP 0 382 259 B1**
(45) Date of publication and mention of the grant of the patent: **01.05.1996**
(21) Application number: 90102612.0
(22) Date of filing: 09.02.1990
(51) Int. Cl.: C12N 1/14, D21H 27/00

(54) **A complex of a fibrous material and fungi and a process for its preparation**
Ein Komplex von Fasermaterial und Pilzen und Verfahren zu seiner Herstellung
Un complexe d'un matériau fibreux et de champignons et son procédé de préparation

(30) Priority: 10.02.1989 JP 32180/89
(43) Date of publication of application: 16.08.1990
(73) Proprietor: AJINOMOTO CO., INC., Tokyo 104 (JP)
(72) Inventor: Yamanaka, Shigeru, c/o Central Res. Laboratories, Kawasaki-ku, Kawasaki-shi Kanagawa-ken (JP); Kikuchi, Reiko, c/o Central Res. Laboratories, Kawasaki-ku, Kawasaki-shi Kanagawa-ken (JP)
(74) Representative: Strehl Schübel-Hopf Groening & Partner

(56) References cited:
- EP-A- 0 095 239
- DE-A- 2 838 751
- DE-A- 2 945 005
- GB-A- 1 495 029
- GB-A- 2 165 865
- US-A- 4 378 431
- TAPPI, vol. 65, no. 6, June 1982, pages 93-96, Atlanta, Georgia, US; L. PILON et al.:
- BIOTECHNOLOGY AND BIOENGINEERING, vol. 24, 1982, pages 2063-2076, New York, US; L. PILON et al.:
- BIOSIS WPI/DERWENT, accession no. 77-16136y (9), 1977, Derwent Publications Ltd., London, GB; & SU - A - 507677 (BEKI BELORUSS KIROV TECHN. INS.) 10.04.1976
- CHEMICAL ABSTRACTS, vol. 76, 18 May 1972, page 106, abstract no. 101496r, Columbus, Ohio, US; M.M. GRINBERG et al.:

## Description

The present invention relates to a novel complex of fibrous materials and fungi which may be obtained by growing fungi in a medium containing fibrous materials thereby bonding the fibrous materials to the fungi.

The complex can be utilized as a constructing or building material such as for fiberboards, heat insulating materials, etc.; acoustic materials such as acoustic diaphragms, speaker cones, etc., base material for fiber-reinforced plastics (FRP); packing materials such as for packaging floppy diskets, for envelopes, etc.; as paper; and the like.

In order to prepare paper from pulp manufactured from wood, a beating step was necessary in the prior art. That is, paper is formed by bonding pulp fibers to each other through hydrogen bonding but the hydrogen bonding between pulp fibers occurs insufficiently so that it is impossible to obtain paper having a satisfactory strength from a practical viewpoint. Accordingly, pulp fibers are fibrillated at this beating step to increase the surface area, whereby the property of bonding fibers to each other by hydrogen bonding is enhanced and as the result, paper can be obtained. However, expensive facilities and large quantities of energy were required for such a beating step. There is not any process for paper-making without requiring this beating step in the prior art.

Furthermore, the necessity of securing the resources of wood in a global scale has been strongly emphasized in recent years. Waste paper is regenerated and used as a new paper-making raw material. However, paper regenerated from waste paper involves the problem that its strength is deteriorated as compared to paper prepared from wood pulp and a part of the fibers constituting the waste paper falls apart at the regeneration step. Therefore, waste paper is utilized by mixing pulp prepared from wood with this waste paper. As such, waste paper has been used as raw material for paper-making, in addition to wood resources. On the other hand, a technique for mixing fungi cultured using industrial waste water or city sewerage with pulp has been disclosed (cf. Japanese Patent Publication No. 57-10280). However, the breaking length of paper thus prepared is merely less than 0.9 km and the strength of the paper is considerably weak as compared to paper ordinarily used and hence, such paper cannot be a substitute for conventional paper.

Further in GB-A-2,165,865, there is disclosed a process which comprises treating fungi with an alkali solution to dissolve chitin or chitosan out, mixing thus treated fungi with other fibers to prepare non-woven fabric and using the non-woven fabric as wound dressings, wet wipes or adsorbents of metal ions. However, the non-woven fabric prepared by the above process does not have sufficient strength as paper, as in the aforesaid product obtained by mixing fungi with pulp. Pilon et. al. (1982), TAPPI, 65, p. 93-96 and Pilon et. al. (1982), Biotech. and Bioeng. XXIV, 2063-2076 disclose a method for improving the sheet strength of paper comprising the partial degradation of cellulose, hemicellulose and lignin of the paper pulp by fungal enzymes.

In Biosys. Derwent no. 77-16136Y (C9), 1977, a similar principle is applied. Cellulose fibers obtained from wood chips are pretreated with a fungal enzymatic medium before sulfite treatment in order to shorten the digestion time.

EP-A-0 095 239 uses Aspergillus terreus in order to degrade lignocellulose in a process directed to the delignification of a lignocellulosic material.

GB-A-1 495 029 relates to a treatment method of waste paper in which the cellulose coating of vegetable parchment paper is degraded by bacterial enzymes.

The present invention aims at solving the problem of preparing paper having a strength comparable to that of paper prepared in a conventional manner, without requiring beating and further in improving the strength of paper regenerated from waste paper to increase the utilization rate of waste paper.

As a result of extensive investigations to solve the problem described above, the present inventors have found that a complex of a fibrous material and fungi can be obtained by allowing fungi to grow in a medium containing a fibrous material and this complex is excellent in its strength and can enhance the utilization rate of waste paper when waste paper is regenerated; etc. and the foregoing problems can thus be solved. The present invention is thus accomplished.

The present invention provides a complex of a fibrous material and fungi, obtainable by cultivating fungi selected from Aspergillus oryzae, Aspergillus sojae and Rhizopus, in a medium containing a fibrous material, which is selected from wood pulp fibers, regenerated cotton and regenerated waste paper. According to a preferred embodiment the fibers are wood pulp fibers, or are derived from regenerated waste paper.

This invention further relates to a process for producing a complex of a fibrous material and fungi, wherein fungi selected from Aspergillus oryzae, Aspergillus sojae and Rhizopus are cultivated in a medium containing a fibrous material which is selected from wood pulp fibers, regenerated cotton and regenerated waste paper and the produced complex is recovered. The above described complex of a fibrous material and fungi may be further subjected to a paper making step.

As the medium there is used, for example, a medium suited for the respective fungi containing organic and inorganic nutrients. The medium is added to the aforesaid fibers as they are or to a slurry of the fibers, the fiber content ranges from 0.05 to 80%. In this case, the concentration of organic nutrients in the medium may be as small as 1/10 to 1/100 of ordinary culture media. The cultivation method may be either static culture or agitated culture. In the case of performing agitation, however, it is desired to gently agitate in such a way that the fungi are not solidified to form pellets. The culture may be carried out at a temperature of 65° C or more, depending on the optimum temperature of the specific fungus desirably at 5 to 65° C for a time period of 0.5 to 10 days. When the culture is performed under the conditions described above, a large number of hyphae grow from the surface of the fibers. Thus, the complex of fibrous material and fungi can be obtained.

In the case of preparing paper using this complex as a raw material, no beating step is required but merely subjecting this complex to paper-making in a conventional manner, paper having a sufficient strength comparable to conventional paper can be prepared. That is, in ordinary paper-making, beaten pulp fibers are used. In this case, the isolated fibrils from the surface of native pulp enhance bonding strength of fibrillated pulp fibers to each other so that a satisfactory strength of the paper can be obtained. On the contrary, when the complex of fibers and fungi obtained by the process of the present invention is subjected to paper-making, the fungi grown on the surface of the fibers mediate the bonding so that it is possible to obtain paper having a strength as strong as that of the ordinary paper prepared from fibrillated pulp fibers.

In preparing paper using waste paper as a raw material, hyphae of the fungi are grown on the pulp fibers by applying the technique of the present invention thereto. By that means, paper having a sufficient strength can be prepared using waste paper alone as a raw material for paper-making.

The process of the present invention is characterized in that hyphae grow from the surface of the fibers by culturing fungi in the presence of the fibers. It is believed that after initiating the culture, the edge of hyphae would bind to the surface by any action to start growing. Consequently, fibers are formed to which a large number of hyphae are bound branch-like. While it is currently unclear which mechanism causes the bonding between the hyphae edge and the fiber surface, it is thought that hyphae would decompose a part of the fibers to form holes or cracks, into which hyphae would permeate. Even though the complex obtained by once bonding hyphae to fibers is put in water and agitated, it is not easy to separate the hyphae and the fibers. Since one end of the hyphae is bound to the fibers as such, a sheet having a high strength can be obtained when the thus obtained complex is subjected to a paper-making process. In this regard, when fungi alone are separately cultured and then mixed with fibers, the bonding between the fibers and the hyphae expected to be formed when fungi are cultured in the presence of the fibers does not occur. Therefore, the strength of the sheet obtained by subjecting a mere mixture of hyphae and fibers to paper-making is lower than that of the sheet obtained by subjecting to paper-making the complex of hyphae and fibers obtained by culturing fungi in the presence of fibers.

As described above, hyphae are allowed to grow on the surface of various fibrous materials to obtain the complex. In the case that the final product is contaminated with components of fungi such as pigments, proteins, etc. which may cause inconvenience, these components may be removed or decomposed after completion of the culture by washing with an acid solution, an alkali solution, a surface active agent, an organic solvent, etc.; or by bleaching.

Hereafter the present invention is described by referrring to the examples below.

### Example 1

### Medium I

| | |
|---|---|
| Hardwood pulp | 2.0 g |
| Yeast extract (Difco Co.) | 0.3 g |
| Malt extract (Difco Co.) | 0.5 g |
| Polypeptone (Daigo Co.,) | 0.5 g |
| Glucose | 1.0 g |
| Tap water | to make 1 liter |
| (1 liter each was separately charged in a shaker flask of 3 liter volume) | |
| pH = 7.0, autoclaved at 120° C for 20 minutes. | |

The pulp was disintegrated with a disintegrator (manufactured by Kumagai Riki Industry Co., Ltd. ). Conditions for disintegration were at room temperature, 3,000 rpm for 3 minutes in medium.

### Medium II

| | |
|---|---|
| Potato dextrose agar (Nissui Pharmaceutical Co., Ltd.) | 39 g |
| Tap water | to make 1 liter |
| (150 ml each was separately charged in a Roux flask of 500 ml volume) | |
| pH 5.6 + 0.1, autoclaved at 120° C for 20 minutes. | |

Fungi (Aspergillus oryzae ATCC 15240) was cultured in medium II at 28° C for 3 to 4 days. The cells were thoroughly suspended by adding thereto 30 ml of 0.9% saline per one Roux flask. The cells were passed through a sterilized cotton cloth to obtain spores alone. The spore suspension was stored at -80°C. The spore suspension was inoculated by 1 ml each per shaker flask filled with medium I. While gently stirring at 60 rpm with a magnetic stirrer, culture was carried out at 28° C for 2 days. Water and spores were removed from the culture solution by sieving through a 200 mesh sieve. Further by repeating washing with pure water, pure white hyphae-containing pulp was obtained. The obtained hyphae-containing pulp was subjected to suction filtration so as not to be heterogeneous and formed into a sheet. The sheet was dried at 105° C to a constant weight and hot-pressed to give a sheet having a uniform thickness.

For control, a sheet prepared from a mixture of fungi grown on pulp-free medium I and pulp and sheets made of pulp alone (two kinds of sheets; one involves a beating step and another involves no beating step) were used. In any case, disintegration of pulp, autoclaving and sheeting step were performed under the same conditions as described above.

Breaking length, folding endurance and dimensional stability of the above sheets were examined.

The dimensional stability was determined by drawing a line on a sheet, wetting the sheet with water, then drying and determining the ratio of the length of the line drawn on the thus treated sheet to the original length. The results are shown in Table 1.

**Table I**

| Sample Sheet | Breaking Length | Folding Endurance | Dimensional Stability |
|---|---|---|---|
| | (km) | (time) | (%) |
| Hyphae was cultured in the presence of pulp. | 5.78 | 1213 | 95 |
| After culturing hyphae, pulp was mixed. | 3.25 | 556 | 88 |
| Pulp alone, no beating | 1.98 | 45 | 75 |
| Pulp alone, with beating | 5.86 | 1009 | 97 |

From the above results, it is noted that when fungi were cultured in the medium containing pulp, the strength was approximately 2.2 times that of pulp alone without beating and approximately 1.7 times that of the mere mixture. The strength of the sheet of the present invention was comparable to that prepared from pulp alone with beating.

### Example 2

### Medium I

| | |
|---|---|
| Yeast extract | 1.5 g |
| Malt extract | 1.5 g |
| Polypeptone | 2.5 g |
| Glucose | 1.0 g |
| Tap water | to make 1 liter |
| pH = 7.0, thermally sterilized at 120° C for 20 minutes. | |

Medium I was sprayed onto autoclaved hardwood pulp and a spore suspension of Aspergillus sojae ATCC 20245 was inoculated thereon followed by culturing at 28° C for 2 days. After completion of the culture, the cells were washed with tap water on a sieve of 200 mesh and then autoclaved at 120° C for 20 minutes. The obtained pulp-hyphae mixture was formed into a web. The web was drawn, glued, dried and wound up on a conveyor belt to prepare a sheet.

For control, a sheet made of pulp alone and a sheet prepared from a mixture of pulp and hyphae in an amount equal to that described above were prepared.

Basis weight, breaking length and folding endurance of the thus prepared sheets and conventional dust paper were examined.

The results are shown in Table 2.

**Table 2**

| Fibrous Material | Hyphae was cultured in the presence of pulp | After culturing hyphae, pulp was mixed | Pulp alone, no beating | Dust Paper |
|---|---|---|---|---|
| Basis weight (g/m) | 70 | 70 | 72 | 71 |
| Breaking length (km) | 7.58 | 6.84 | 2.43 | 7.86 |
| Folding endurance (time) | 2630 | 980 | 58 | 1035 |

From the above results, it is noted that by culturing fungi on pulp, clean wiper having excellent breaking length and folding endurance was obtained.

### Example 3

Waste newspaper was cut into 1 x 3 cm and 3 g of the resulting pieces were mixed with 1000 ml of water together with 0.2 g of sodium hydroxide and 0.5 g of sodium silicate. After the addition of sodium hypochlorite to the mixture in an amount of available chlorine of 0.02%, the mixture was disintegrated with a disintegrator (manufactured by Kumagai Riki Industry Co., Ltd. ). Conditions for the disintegration were room temperature and 3,000 rpm for 20 minutes. The resulting disintegrated solution was concentrated to a solid content of 7% by suction filtration. The concentrate was resuspended in 1000 ml of water and the suspension was neutralized with hydrochloric acid. The suspension was again concentrated to a solid content of 7% by suction filtration and 1000 ml of water was further added to the concentrate to give a pulp slurry.

Medium components were added to 1000 ml of this pulp slurry in final concentrations of 30 g/l of yeast extract, 30 g/l of malt extract, 50 g/l of polypeptone and 100 g/l of glucose, pH 7.0 (adjusted with hydrochloric acid and sodium hydroxide). Then 1 x 10⁴/ml of Aspergillus sojae ATCC 20245 spores were inoculated on the pulp slurry. By culturing at 28° C for 3 days with gentle agitation, a slurry was obtained (culture slurry).

Next, the same fungi was inoculated on a medium composed of 30 g of yeast extract, 30 g of malt extract, 50 g of polypeptone and 100 g of glucose, 1.0 liter of tap water, adjusted to pH 7.0, in the same concentration. By culturing at 28° C for 3 days with gentle agitation, a hyphae was obtained. This hyphae was mixed with the concentrate obtained by concentrating the aforesiad pulp slurry obtained by disintegration of 30 g of conventional newspaper to a concentration of 7% with agitation to give a slurry (slurry mixture).

The aforesaid 3 kinds of slurry: culture slurry, slurry mixture and pulp slurry, were filtered through an 80 mesh sieve having a diameter of 16 cm and further washed twice with 1 liter of hot water at 55° C. The resulting filtrate was squeezed out under pressure to form wet mats. The mats were dried under tension to give sheets. Properties of the 3 sheets were examined. The results are shown in Table 3.

**Table 3**

| Kind of Sheet | Breaking Length (km) |
|---|---|
| Sheet prepared from culture slurry | 2.54 |
| Sheet prepared from slurry mixture | 1.66 |
| Sheet prepared from pulp slurry | 1.21 |
| Recovered used newspaper | 2.25 |

The sheet prepared from the pulp slurry, and the sheet prepared from the slurry mixture of fungi hyphae and pulp slurry cannot be used as raw materials for making newspaper since the breaking lengths thereof were poor as compared to the newspaper. However, the sheet prepared from the culture slurry obtained by culturing fungi and pulp slurry at the same time can be re-used as newspaper since the sheet obtained is stronger than the newspaper.

### Example 4

The 3 kinds of sheets prepared in Example 3 were cut into 1 x 3 cm, respectively, and the resulting pieces were disintegrated in the same manner as described above. Sheets were prepared from the 3 kinds of slurry obtained by disintegration and the breaking length was measured. The sheets were again cut and disintegrated to form sheets and the breaking length was measured. These operations were repeated 3 times and 3 measurement values of the breaking length in total were obtained. The results are shown in Table 4.

**Table 4**

| Kind of Sheet | Breaking Length (km) | | | |
|---|---|---|---|---|
| (Sheet prepared in Example 3) | 0*¹ | 1st | 2nd | 3rd |
| Sheet prepared from culture slurry | 2.54* | 2.49 | 2.30 | 2.28 |
| Sheet prepared from slurry mixture | 1.66* | 1.16 | 0.99 | 0.75 |
| Sheet prepared from pulp slurry | 1.21* | 0.95 | 0.88 | 0.73 |

| | | | | |
|---|---|---|---|---|
| *1: Times of regeneration of sheet | | | | |
| *2: Breaking length in Example 3 | | | | |

When disintegration and paper-making are repeated using as raw materials the sheet prepared from the pulp slurry or the sheet prepared from the slurry mixture of fungi hyphae and pulp slurry, the breaking length of the sheets are rapidly reduced by repeating disintegration and paper-making. To the contrary, when disintegration and paper-making are repeated using as raw materials the sheet prepared from the culture slurry obtained by culturing fungi and pulp slurry together, the breaking length of the sheet was not reduced appreciably.

### Effects of the Invention:

According to the present invention, paper having a high strength can be prepared without requiring any beating step, in the paper-making industry. Furthermore, in the case of using waste paper as raw materials for paper-making, paper having a sufficient strength can be prepared from waste paper alone, resulting in saving raw materials for paper-making and thus contributing to protection of natural wood resources.

## Claims

1. A complex of a fibrous material and fungi, obtainable by cultivating fungi selected from Aspergillus oryzae, Aspergillus sojae and Rhizopus, in a medium containing a fibrous material, which is selected from wood pulp fibers, regenerated cotton and regenerated waste paper.

2. A complex according to claim 1, wherein the fibers are wood pulp fibers.

3. A complex according to claim 1 or claim 2, wherein the fibers are derived from regenerated waste paper.

4. A process for producing a complex of a fibrous material and fungi, wherein fungi selected from Aspergillus oryzae, Aspergillus sojae and Rhizopus are cultivated in a medium containing a fibrous material which is selected from wood pulp fibers, regenerated cotton and regenerated waste paper and the produced complex is recovered.

5. A process according to claim 4, wherein the fibers are according to claim 2 or 3.

6. A process according to claim 5, wherein the complex is further subjected to a paper making step.

## Patentansprüche

1. Komplex aus einer faserförmigen Substanz und Pilzen, der durch Kultivieren von unter Aspergillus oryzae, Aspergillus sojae und Rhizopus ausgewählten Pilzen in einem Medium, das eine faserförmige Substanz enthält, die ausgewählt ist unter Zellstoffasern, regenerierter Baumwolle und regeneriertem Altpapier, erhältlich ist.

2. Komplex gemäß Anspruch 1, worin die Fasern Zellstofffasern sind.

3. Komplex gemäß Anspruch 1 oder 2, worin die Fasern aus regeneriertem Altpapier stammen.

4. Verfahren zur Herstellung eines Komplexes einer faserförmigen Substanz und Pilzen, wobei unter Aspergillus oryzae, Aspergillus sojae und Rhizopus ausgewählte Pilze in einem Medium, das eine faserförmige Substanz enthält, die ausgewählt wird unter Zellstoffasern, regenerierter Baumwolle und regeneriertem Altpapier, kultiviert werden, und der produzierte Komplex gewonnen wird.

5. Verfahren gemäß Anspruch 4 mit Fasern gemäß Anspruch 2 oder 3.

6. Verfahren gemäß Anspruch 5, worin der Komplex ferner einer Papierherstellungsstufe unterworfen wird.

## Revendications

1. Complexe d'une matière fibreuse et de champignons, qu'il est possible d'obtenir par culture de champignons sélectionnés parmi Aspergillus oryzae, Aspergillus sojae et Rhizopus, dans un milieu contenant une matière fibreuse qui est sélectionnée parmi des fibres de pâte de bois, du coton recyclé et des vieux papiers recyclés.

2. Complexe selon la revendication 1, dans lequel les fibres sont des fibres de pâte de bois.

3. Complexe selon la revendication 1 ou 2, dans lequel les fibres proviennent de vieux papiers recyclés.

4. Procédé de production d'un complexe d'une matière fibreuse et de champignons, dans lequel des champignons sélectionnés parmi Aspergillus oryzae, Aspergillus sojae et Rhizopus sont cultivés dans un milieu contenant une matière fibreuse qui est sélectionnée parmi des fibres de pâte de bois, du coton recyclé et des vieux papiers recyclés, et le complexe produit est récupéré.

5. Procédé selon la revendication 4, dans lequel les fibres sont selon la revendication 2 ou 3.

6. Procédé selon la revendication 5, dans lequel le complexe est soumis en outre à une étape de fabrication de papier.
